(19) **Europäisches Patentamt / European Patent Office / Office européen des brevets**

(11) **EP 4 603 470 A1**

(12) # EUROPEAN PATENT APPLICATION

(43) Date of publication:
20.08.2025 Bulletin 2025/34

(21) Application number: 24382166.7

(22) Date of filing: 19.02.2024

(51) International Patent Classification (IPC):
*C07C 5/333* (2006.01)        *C07C 5/48* (2006.01)
*C07C 11/02* (2006.01)

(52) Cooperative Patent Classification (CPC):
(C-Sets available)
**C07C 5/48; B01J 23/005; B01J 23/78; B01J 23/94;**
**B01J 35/40; B01J 35/615; B01J 35/635;**
**B01J 37/0201; B01J 37/0207; B01J 38/02;**
**B01J 38/12; C07C 5/3332;** B01J 2235/00;
B01J 2235/15; C07C 2521/04;        (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Dow Global Technologies LLC**
**Midland, MI 48674 (US)**

(72) Inventors:
• **MILLAR, Dean M.**
**Midland, 48674 (US)**
• **LIU, Chuncheng**
**4542 NM Terneuzen (NL)**

• **KIRILIN, Alexey**
**4542 NM Terneuzen (NL)**
• **RIVERA TORRENTE, Miguel**
**28760 Tres Cantos (ES)**
• **PARASTAEV, Alexander**
**4542 NM Terneuzen (NL)**
• **MALEK, Andrzej**
**Midland, 48674 (US)**
• **DINH, Kimberly**
**Midland, 48674 (US)**
• **BLANN, Kevin**
**Freeport, 77541 (US)**

(74) Representative: **Elzaburu S.L.P.**
**Edificio Torre de Cristal**
**Paseo de la Castellana 259 C, planta 28**
**28046 Madrid (ES)**

(54) **METHODS FOR CONVERTING ALKANES TO ALKENES USING STEAM TOLERANT DEHYDROGENATION CATALYSTS WITH SPINEL STRUCTURES**

(57)    A method for converting alkanes to alkenes includes introducing a feed stream comprising alkanes to a reaction zone comprising steam and a dehydrogenation catalyst, the dehydrogenation catalyst comprising a component having a spinel structure and a metal catalyst component selected from the group consisting of chromium, gallium, iron, cobalt, and combinations thereof. The method further includes converting at least a portion of the alkanes to alkenes, thereby yielding a product stream comprising alkanes, alkenes, and hydrogen, wherein the dehydrogenation catalyst does not require a gaseous oxidant in the feed stream or as a co-feed to catalyze conversion of alkanes to alkenes.

(52) Cooperative Patent Classification (CPC): (Cont.)
C07C 2521/06; C07C 2523/02; C07C 2523/06;
C07C 2523/08; C07C 2523/26; C07C 2523/745;
C07C 2523/75; C07C 2523/78

C-Sets
**C07C 5/3332, C07C 11/04;**
**C07C 5/48, C07C 11/04**

## Description

### TECHNICAL FIELD

The present disclosure relates to methods for efficiently converting various alkanes to alkenes. In particular, the present disclosure relates to methods for converting alkanes to alkenes using dehydrogenation catalysts that tolerate steam, and more particularly to methods of using the dehydrogenation catalysts having a spinel structure to achieve a high conversion of alkanes to alkenes in the presence of steam.

### BACKGROUND

Alkenes are used for a wide range of industrial applications, including producing plastics, fuels, and various downstream chemicals. Such alkenes include $C_2$ to $C_4$ materials, including ethene, propene, and butenes (also commonly referred to as ethylene, propylene, and butylenes, respectively). A variety of processes for producing these alkenes have been developed, including petroleum cracking and various synthetic processes.

One such process for producing alkenes is alkane dehydrogenation. Conventional alkane dehydrogenation is endothermic and equilibrium limited. Therefore, to reach economically feasible levels of alkane-to-alkene conversion, conventional alkane dehydrogenation necessitates the use of low pressures to shift the equilibrium toward producing products and high temperatures, often in excess of 800 °C, to provide thermal energy. Additionally, conventional alkane dehydrogenation processes suffer from additional undesirable radical chemistry that may produce coke as a byproduct. The formation of coke may cause blockages, which may require periodic process shutdowns for decoking operations.

Maintaining the low pressures and high temperatures necessary for economically feasible alkane-to-alkene conversion can be expensive. Accordingly, a need exists for methods and catalytic systems with high alkene selectivity that operate at higher pressures and lower temperatures while reaching economically feasible levels of alkane-to-alkene conversion.

### SUMMARY

Embodiments of the present disclosure address these and other needs by the methods of converting alkanes to alkenes using dehydrogenation catalysts that are capable of performing dehydrogenation chemistry in the presence of steam, and particularly dehydrogenation catalysts having a spinel structure. A dehydrogenation catalyst, as described herein, comprises a component having a spinel structure and a metal catalyst selected from the group consisting of chromium, gallium, iron, cobalt, and combinations thereof. This dehydrogenation catalyst may then be used in dehydrogenation processes to convert alkanes to alkenes. In embodiments, the dehydrogenation catalyst may be able to catalyze the conversion of alkanes to alkenes in the presence of steam.

According to one or more embodiments of the present disclosure, a method for converting alkanes to alkenes may comprise introducing a feed stream comprising alkanes to a reaction zone comprising steam and a dehydrogenation catalyst. The dehydrogenation catalyst may comprise a component having a spinel structure and a metal catalyst component selected from the group consisting of chromium, gallium, iron, cobalt, and combinations thereof, and converting at least a portion of the alkanes to alkenes, thereby yielding a product stream comprising alkanes, alkenes, and hydrogen. The dehydrogenation catalyst does not require a gaseous oxidant in the feed stream or as a co-feed to catalyze conversion of alkanes to alkenes.

Additional features and advantages will be set forth in the detailed description that follows and, in part, will be readily apparent to those skilled in the art from that description or recognized by practicing the embodiments described herein, including the detailed description which follows in addition to the claims.

It is to be understood that both the foregoing general description and the following detailed description describe various embodiments and are intended to provide an overview or framework for understanding the nature and character of the claimed subject matter.

### DETAILED DESCRIPTION

Reference will now be made in detail to embodiments of dehydrogenation catalysts, and more particularly, dehydrogenation catalysts that tolerate steam, and methods of using the dehydrogenation catalysts to convert alkanes to alkenes in the presence of steam.

As used herein, "steam conditions" refers to reaction conditions where at least some amount of steam is present. For example, a reaction might take place under 20 volume percent (v.%) steam conditions wherein 20% of the gas volume of the reaction section would be filled with steam. The steam that leads to steam conditions may come from any source. For example, the steam that leads to steam conditions may be generated in-situ by selective hydrogen combustion materials.

As used herein, "dehydrogenation" refers to a chemical process by which hydrogen is chemically removed from a compound. For example, ethane may undergo dehydrogenation to be converted to ethylene. As used herein, "dehydrogenation catalyst(s)" refers to any substance that increases the rate of a dehydrogenation reaction without itself undergoing any permanent chemical change. As used herein, "promoted dehydrogenation catalyst(s)" refers to a catalyst that has had an amount of activator (also commonly referred to as catalytic promoter) added to the catalyst. The activator may increase the catalytic efficiency by improving catalytic selectivity, catalytic activity, or combinations thereof. When the activator improves both catalytic selectivity and catalytic activity, an improved product yield will result. For example, silicon might be added to a dehydrogenation catalyst to increase the dehydrogenation catalyst's catalytic efficiency. As used herein, "background dehydrogenation activity" refers to the dehydrogenation activity that occurs in the presence of inert material instead of a catalyst measured under the same process conditions. For example, a dehydrogenation catalyst may have an activity equal to 1.1 times background dehydrogenation activity when the dehydrogenation catalyst performs dehydrogenation at a conversion rate equal to 1.1 times the dehydrogenation activity of quartz chips under the same process conditions.

As used herein, "alkane(s)" refers to any series of hydrocarbon molecules that consist of carbon-carbon single bonds and where the carbon structure is saturated with hydrogen. Ethane, propane, and butane are examples of alkanes. As used herein, "alkene(s)" refers any series of hydrocarbon molecules, where at least two of the carbon atoms are not saturated with hydrogen and share a double bond. Ethylene, propylene, 1-butene, trans-2-butene, and *cis*-2-butene are examples of alkenes. Alkenes include dienes, which are hydrocarbons where at least two sets of two of the carbon molecules, that may or may not be adjacent to each other, are not saturated with hydrogen and share a double bond.

The use of dehydrogenation catalysts is known in the field of hydrocarbon products, such as plastics, fuels, and various downstream chemicals. For example, the Catofin propane dehydrogenation processes from Lummus Technology and the Oleflex propane dehydrogenation processes from Honeywell employ $Cr/Al_2O_3$ and Pt-Sn-based dehydrogenation catalysts, respectively. Additionally, spinel catalysts are known for use in oxidative dehydrogenation of alkanes. In the oxidative dehydrogenation processes, alkanes are typically co-fed with a gaseous oxidant such as oxygen, air, carbon dioxide, or nitrogen oxides, thus shifting the equilibrium constraint of the dehydrogenation reaction.

Oxidative dehydrogenation occurs at the surface of the catalyst by a reaction of alkane and oxidant and generates water. However, the presence of water and alkanes at high temperatures can lead to reduced alkene selectivity through oxidation and reforming reactions that yield methane and carbon oxide products such as carbon monoxide and carbon dioxide. Furthermore, many oxidative dehydrogenation catalysts, when used in the absence of a gaseous oxidant in the feed stream or as a co-feed, exhibit significantly reduced activity in the presence of steam. Thus, not every oxidative dehydrogenation catalyst is a steam tolerant alkane dehydrogenation catalyst. In contrast, the dehydrogenation catalysts disclosed and described herein exhibit steam tolerance, even in the absence of a gaseous oxidant in the feed stream or as a co-feed. The preparation and composition of such dehydrogenation catalysts used in embodiments are discussed below.

In embodiments, a method for converting alkanes to alkenes comprises introducing a feed stream comprising alkanes to a reaction zone comprising steam and a dehydrogenation catalyst. The dehydrogenation catalyst comprises a component having a spinel structure and a metal catalyst component selected from the group consisting of chromium, gallium, iron, cobalt, and combinations thereof. The method further comprises converting at least a portion of the alkanes to alkenes, thereby yielding a product stream comprising alkanes, alkenes, and hydrogen. The dehydrogenation catalyst does not require a gaseous oxidant in the feed stream or as a co-feed to catalyze conversion of alkanes to alkenes.

In embodiments, the metal catalyst component may be part of the spinel structure. The dehydrogenation catalyst may comprise a metal catalyst component in any suitable oxidation state. According to embodiments, the chromium may have an oxidation state of +2, +3, +4, +5, +6, or combinations thereof. In embodiments, the dehydrogenation catalyst may comprise chromium having a single oxidation state or the dehydrogenation catalyst may comprise chromium having different oxidation states. According to embodiments, the gallium may have an oxidation state of +1, +3, or combinations thereof. In embodiments, the dehydrogenation catalyst may comprise gallium having a single oxidation state or the dehydrogenation catalyst may comprise gallium having different oxidation states. According to embodiments, the iron may have an oxidation state of +2, +3, +4, +6, or combinations thereof. In embodiments, the dehydrogenation catalyst may comprise iron having a single oxidation state or the dehydrogenation catalyst may comprise iron having different oxidation states. According to embodiments, the cobalt may have an oxidation state of +2, +3, or combinations thereof. In embodiments, the dehydrogenation catalyst may comprise cobalt having a single oxidation state or the dehydrogenation catalyst may comprise cobalt having different oxidation states.

In embodiments, the dehydrogenation catalyst may comprise a spinel structure. As used herein, "spinel structure" may refer to a specific arrangement of atoms in a crystal lattice. A standard spinel structure may be represented by $AB_2O_4$, where A and B are metal cations, and O represents oxygen. The spinel structure has a cubic close-packed arrangement of oxygen ions, and the metal cations occupy some of the tetrahedral and octahedral sites within this oxygen lattice. The general formula $AB_2O_4$ can be understood by considering two types of metal cations, A and B. The A cations occupy tetrahedral sites, and the B cations occupy octahedral sites in the crystal lattice. The arrangement of these metal cations within the oxygen lattice gives rise to the spinel structure. As used herein, "spinel structure" may refer to an inverse spinel structure, where distribution of cations is altered compared to the standard spinel. An inverse spinel structure may be represented by $A_2BO4$, where A and B are metal cations, and O represents oxygen. In the inverse case, the A cations occupy both octahedral and tetrahedral sites, while the B cations are located solely in octahedral sites. This inversion of cations leads to a different arrangement of atoms within the crystal lattice.

In embodiments, A may be a metal selected from the group consisting of magnesium (Mg), calcium (Ca), zinc (Zn), and combinations thereof, and B may be selected from the group consisting of chromium (Cr), gallium (Ga), cobalt (Co), iron (Fe), zinc (Zn), titanium (Ti), and combinations thereof. In embodiments, B may be zinc (Zn) and titanium (Ti) in a 1:1 atomic ratio.

In embodiments, a solvothermal method for forming a dehydrogenation catalyst may comprise obtaining precursor materials containing the desired metal cations (A and B) for the spinel structure. The precursor materials are dissolved in a selected solvent or solvent mixture, creating a homogeneous solution. Examples of precursor materials include, but are not limited to, nitrate, acetate, and alkoxide. Solvents include, but are not limited to, deionized water, methanol, and ethanol. The solution is then sealed in an autoclave and subjected to elevated temperatures and pressures. This promotes accelerated chemical reactions, leading to the formation of the spinel structure. After the solvothermal reaction, the catalyst is isolated, washed to remove impurities, and then dried to obtain the final spinel powder or solid material. The thermal treatment of the catalyst may occur at temperatures greater than 750 °C for at least 1 hour in air.

In other embodiments, a co-precipitation method for preparation of mixed-metal spinel catalysts may include obtaining a precursor solution containing salts or compounds of the desired metal cations (A and B) is mixed with a base, such as sodium hydroxide or ammonia. The simultaneous addition of the base initiates the precipitation of metal hydroxides, leading to the formation of a mixed metal hydroxide precursor. This mixed precursor is then separated from the solution through filtration, washed to remove impurities, and subsequently dried to obtain a powder. The dried precursor powder undergoes calcination at temperatures greater than 750 °C for at least 1 hour in air. During calcination, the mixed metal hydroxide undergoes dehydration and oxidation, resulting in the formation of the desired spinel structure.

In embodiments, the dehydrogenation catalyst may comprise a promoter selected from the group consisting of cobalt (Co), chromium (Cr), iron (Fe), platinum (Pt), tin (Sn), boron (B), silicon (Si), zirconium (Zr), and combinations thereof. In one or more embodiments, the promoter may comprise cobalt (Co), chromium (Cr), iron (Fe) or combinations thereof. The promoters may enhance catalytic performance by increasing ethylene selectivity and reducing reforming product selectivity. In embodiments, the promoter may be within the spinel structure as $A_{1.0}B_xAl_{2-x}O_4$, where the total number of B atoms and Al atoms is equal to 2. In embodiments, the presence of a promoter in the dehydrogenation catalyst may enhance the alkane dehydrogenation rate, reduce the inhibition effect of steam on dehydrogenation, reduce the selectivity of by-products (such as reforming products like carbon monoxide, carbon dioxide, and methane), coke, and other heavy byproducts, enhance the yield of ethylene, and combinations of the foregoing.

In embodiments, the metal catalyst component may be supported by the spinel structure $AB_2O_4$ or by the inverse spinel structure $A_2BO_4$. The spinel-type support may be impregnated with an active metal such as chromium, gallium, iron, cobalt, manganese, and combinations thereof. A may be a metal selected from the group consisting of magnesium (Mg), calcium (Ca), zinc (Zn), and combinations thereof, and B may be selected from the group consisting of aluminum (Al), gallium (Ga) and combinations thereof. In embodiments, B may be zinc (Zn) and titanium (Ti) in a 1:1 atomic ratio.

An incipient wetness impregnation method may be utilized to impregnate a metal component on a spinel structure. In embodiments, a spinel-type support, such as $MgAl_2O_4$, is contacted with a solution of metal precursors to fill the incipient wetness volume of the solid support. The spinel structure absorbs the solution, facilitating the impregnation of the metal precursor into its pores. After impregnation, the catalyst undergoes a drying step to remove the solvent, preventing unwanted by-products and ensuring proper adhesion of the metal precursor to the support. Calcination may follow at temperatures greater than 750 °C for at least 1 hour in air, promoting the decomposition of the metal precursor and the formation of the desired supported metal oxide phase. The resulting supported metal oxide catalyst exhibits enhanced surface area and reactivity due to the porous nature of the support material.

In one or more embodiments, a method for converting alkanes to alkenes may comprise introducing a feed stream comprising alkanes to a reaction zone comprising steam and a dehydrogenation catalyst. It should be understood that steam may not be present when the feed stream is first introduced into the reaction zone, but as the conversion of alkanes to alkenes proceeds, steam is produced and may be present within the reaction zone as the feed stream is introduced to the reaction zone. The feed stream may comprise $C_2$-$C_4$ alkanes, such as, but not limited to, ethane, propane, butanes, or combinations thereof. In embodiments, the feed stream may be contacted with the dehydrogenation catalyst for a controlled time of exposure. The controlled time of exposure may be selected based on the desired catalyst to feed stream mass to mass ratio. In embodiments, the controlled time of exposure may be from 5 seconds (sec) to 1 hour (h). In embodiments, the controlled time of exposure may be from 5 seconds (sec) to 1 h, from 10 sec to 30 minutes (min), from 15 sec to 15 min, from 20 sec to 10 min, from 25 sec to 5 min, from 25 sec to 30 sec, from 30 sec to 1 min, or any combination thereof.

In one or more embodiments, the reaction zone may be a zone inside a reactor adapted to allow the feed stream to be contacted with the dehydrogenation catalyst. In one or more embodiments, the reactor may be a fixed-bed reactor, including but not limited to a dual tube fixed-bed reactor. In embodiments, the reactor may be a circulating fluidized bed reactor. In embodiments, the reaction zone may be two or more reactors in series or parallel, and each reactor in series or parallel may be the same type of reactor as other reactors in the series, or may be a different type of reactor from other reactors in the series. In embodiments, the reaction zone may house a material that converts gaseous hydrogen to water.

In one or more embodiments, a method for converting alkanes to alkenes may comprise converting at least a portion of the alkanes to alkenes, thereby yielding a product stream comprising alkanes, alkenes, and hydrogen. In embodiments, the product stream may comprise ethylene, propylene, butylene, hydrogen, or combinations thereof. In embodiments, the product stream may comprise ethane, propane, butane, ethylene, propylene, butylene, hydrogen, or combinations thereof.

In one or more embodiments, at least a portion of the hydrogen in the product stream is combusted and yields water. In embodiments, the water may be in the form of steam. In embodiments, the steam may comprise gaseous water, liquid water, aerosolized water, or combinations thereof. Because of this hydrogen combustion, water-such as steam-will be present in the reaction zone during dehydrogenation of the alkanes in the feed stream. As mentioned above, many oxidative dehydrogenation catalysts lose conversion and selectivity when they are exposed to water and require a significant amount of oxidative gas to offset the loss of conversion and selectivity. However, the dehydrogenation catalysts disclosed and described herein retain catalytic activity in the presence of water and retain all or some of their selectivity and conversion when the hydrogen is combusted and forms water. Therefore, the catalysts disclosed and described herein can operate in the presence of water without the addition of oxidative gas.

In one or more embodiments, the dehydrogenation catalyst may have an alkene selectivity of greater than or equal to 40 carbon mole percent (Cmol%), greater than or equal to 45 Cmol%, greater than or equal to 50 Cmol%, greater than or equal to 55 Cmol%, greater than or equal to 65 Cmol%, greater than or equal to 75 Cmol%, greater than or equal to 85 Cmol%, greater than or equal to 95 Cmol%, greater than or equal to 97 Cmol%, greater than or equal to 98 Cmol%, or greater than or equal to 99 Cmol%.

In one or more embodiments, the dehydrogenation catalyst comprises a conversion rate of greater than or equal to 1.1 times background dehydrogenation activity. In embodiments, the dehydrogenation catalyst comprises a conversion rate of greater than or equal to 1.1 times, 1.5 times, 2 times, 3 times, 4 times, 5 times, or 10 times background dehydrogenation activity.

In one or more embodiments, the dehydrogenation catalyst retains at least some dehydrogenation activity above background dehydrogenation activity under greater than or equal to 20 v.% steam conditions based on a total volume of gaseous components in the reaction zone. In embodiments, the dehydrogenation catalyst may retain at least 1%, at least 5%, at least 10% or even at least 20% dehydrogenation activity under greater than or equal to 5 v.%, 10 v.%, 15 v.%, 20 v.%, 25 v.%, 30 v.%, 35 v.%, 40 v.%, 45 v.%, or 50 v.% steam conditions based on a total volume of gaseous components in the reaction zone.

In embodiments, the dehydrogenation catalyst comprises a dehydrogenation activity of greater than or equal to 1.1 times, 1.5 times, 2 times, 3 times, 4 times, 5 times, or 10 times background dehydrogenation activity under greater than or equal to 20 v.%, 25 v.%, 30 v.%, 35 v.%, 40 v.%, 45 v.%, 50 v.%, 55 v.%, 60 v.%, or 65 v.% steam conditions based on a total volume of gaseous components in the reaction zone.

In embodiments, the dehydrogenation catalyst may comprise from 1 wt.% to 30 wt.% of the metal catalyst component. In embodiments, the dehydrogenation catalyst may comprise greater than 1 wt.%, greater than or equal to 5 wt.%, greater than 10 wt.%, or even greater than 15 wt.% of the metal catalyst component. In embodiments, the dehydrogenation catalyst may comprise less than or equal to 30 wt.%, less than or equal to 25 wt.%, less than or equal to 20 wt.%, or even less than or equal to 15 wt.%. In embodiments, the dehydrogenation catalyst may comprise greater than or equal to 1 wt.% and less than or equal to 30 wt.%, greater than or equal to 1 wt.% and less than or equal to 25 wt.%, greater than or equal to 1 wt.% and less than or equal to 20 wt.%, greater than or equal to 1 wt.% and less than or equal to 15 wt.%, greater than or equal to 1 wt.% and less than or equal to 10 wt.%, greater than or equal to 1 wt.% and less than or equal to 5 wt.%, greater than or equal to 5 wt.% and less than or equal to 30 wt.%, greater than or equal to 5 wt.% and less than or equal to 25 wt.%, greater than or equal to 5 wt.% and less than or equal to 20 wt.%, greater than or equal to 5 wt.% and less than or equal to 15 wt.%, greater than or equal to 5 wt.% and less than or equal to 10 wt.%, greater than or equal to 10 wt.% and less than or equal to 30 wt.%, greater than or equal to 10 wt.% and less than or equal to 25 wt.%, greater than or equal to 10 wt.% and less than or equal to 20 wt.%, greater than or equal to 10 wt.% and less than or equal to 15 wt.%, greater than or equal to 15 wt.% and less than or equal to 30 wt.%, greater than or equal to 15 wt.% and less than or equal to 25 wt.%, greater than or equal to 15 wt.% and less than or equal to 20 wt.%, greater than or equal to 20 wt.% and less than or equal to 30 wt.%, greater than or equal to 20 wt.% and less than or equal to 25 wt.%, or even greater than or equal to 25 wt.% and less than or equal to 30 wt.% of the metal catalyst component, or any and all sub-ranges formed by these endpoints.

In embodiments, the dehydrogenation catalyst may comprise from 70 wt.% to 99 wt.% of the spinel component. In embodiments, the dehydrogenation catalyst may comprise greater than or equal to 70 wt.%, greater than or equal to 75 wt.%, greater than or equal to 80 wt.%, or even greater than or equal to 85 wt.% of the spinel component. In embodiments, the dehydrogenation catalyst may comprise less than or equal to 99 wt.%, less than or equal to 95 wt.%, less than or equal to 90 wt.%, or even less than or equal to 85 wt.% of the spinel component. In embodiments, the dehydrogenation catalyst may comprise greater than or equal to 70 wt.% and less than or equal to 99 wt.%, greater than or equal to 70 wt.% and less than or equal to 95 wt.%, greater than or equal to 70 wt.% and less than or equal to 90 wt.%, greater than or equal to 70 wt.% and less than or equal to 85 wt.%, greater than or equal to 70 wt.% and less than or equal to 80 wt.%, greater than or equal to 70 wt.% and less than or equal to 75 wt.%, greater than or equal to 75 wt.% and less than or equal to 99 wt.%, greater than or equal to 75 wt.% and less than or equal to 95 wt.%, greater than or equal to 75 wt.% and less than or equal to 90 wt.%, greater than or equal to 75 wt.% and less than or equal to 85 wt.%, greater than or equal to 75 wt.% and less than or equal to 80 wt.%, greater than or equal to 80 wt.% and less than or equal to 99 wt.%, greater than or equal to 80 wt.% and less than or equal to 95 wt.%, greater than or equal to 80 wt.% and less than or equal to 90 wt.%, greater than or equal to 80 wt.% and less than or equal to 85 wt.%, greater than or equal to 85 wt.% and less than or equal to 99 wt.%, greater than or equal to 85 wt.% and less than or equal to 95 wt.%, greater than or equal to 85 wt.% and less than or equal to 90 wt.%, greater than or equal to 90 wt.% and less than or equal to 99 wt.%, greater than or equal to 90 wt.% and less than or equal to 95 wt.%, or even greater than or equal to 95 wt.% and less than or equal to 99 wt.% of the spinel component, or any and all sub-ranges formed by these endpoints.

In one or more embodiments, the method for converting alkanes to alkenes may further comprise contacting the feed stream comprising alkanes with at least one other catalyst. In embodiments, the at least one other catalyst may comprise a selective hydrogen combustion material. For example, oxygen-carrier materials such as those disclosed in U.S. App. No. 62/725,504, entitled "METHODS OF PRODUCING HYDROGEN-SELECTIVE OXYGEN-CARRIER MATERIALS," filed on, August 31, 2018, and U.S. App. No. 62/725,508, entitled "HYDROGEN-SELECTIVE OXYGEN-CARRIER MATERIALS AND METHODS OF USE," filed on, August 31, 2018, are contemplated as suitable for the presently disclosed processes, and the teachings of these references are incorporated by reference herein. In one or more additional embodiments, the oxygen-carrier material may include those of U.S. Pat. No. 5,430,209, U.S. Pat. No. 7,122,495, and/or WO 2018/232133, each of which are incorporated by reference in their entireties.

In embodiments, the selective hydrogen combustion material may be a promoted selective hydrogen combustion material. In embodiments, the dehydrogenation catalyst and the selective hydrogen combustion material are both present in the reaction zone. In embodiments, the dehydrogenation catalyst and the selective hydrogen combustion material may be present in a mass to mass ratio of from 10:1 to 1:10. In embodiments, the dehydrogenation catalyst and the selective hydrogen combustion material may be present in a mass to mass ratio of from 10:1 to 1:10, from 10:1 to 1:10, from 2:1 to 1:10, from 1:1 to 1:10, from 10:1 to 1:5, from 10:1 to 1:5, from 2:1 to 1:5, from 1:1 to 1:5, from 10:1 to 1:2, from 10:1 to 1:2, from 2:1 to 1:2, from 1:1 to 1:2, from 10:1 to 1:1, from 10:1 to 1:1, or from 2:1 to 1:1. In embodiments, the dehydrogenation catalyst and the selective hydrogen combustion material may be in contact with each other. In embodiments, the dehydrogenation catalyst and the selective hydrogen combustion material may have been mixed or otherwise combined prior to being placed in the reaction zone. In embodiments, the dehydrogenation catalyst and the selective hydrogen combustion material may be mixed in the reaction zone. In embodiments, the dehydrogenation catalyst and the selective hydrogen combustion material may be separate.

As mentioned above, many conventional alkane dehydrogenation processes, such as oxidative dehydrogenation processes, require the use of gaseous oxidants such as oxygen, air, carbon dioxide, or nitrogen oxides in the feed stream or as a co-feed. The term "gaseous oxidant(s)" may refer to a substance or substances other than water that may oxidize hydrogen. However, in one or more embodiments of the present disclosure, the dehydrogenation catalyst maintains the conversion of alkanes to alkenes without the presence of a gaseous oxidant in the feed stream or as a co-feed. In some embodiments, the dehydrogenation catalyst maintains the conversion of alkanes to alkenes with the presence of only a small amount of a gaseous oxidant in the feed stream or as a co-feed. In some embodiments, the dehydrogenation catalyst maintains the conversion of alkanes to alkenes with the presence of less than 5 v.%, less than 4 v.%, less than 3 v.%, less than 2 v.%, less than 1 v.%, less than 0.5 v.%, less than 0.25 v.%, or less than 0.1 v.% gaseous oxidant in the feed stream or as a co-feed.

In one or more embodiments, the dehydrogenation catalyst and the feed stream have a mass to mass ratio that is from 5:1 to 200:1. This mass ratio is defined as the ratio between the feed rate of catalyst to the reaction zone and the feed rate of alkane to the reaction zone. In embodiments, the dehydrogenation catalyst and the feed stream have a mass to mass ratio that is from 5:1 to 200:1, from 10:1 to 200:1, from 25:1 to 200:1, from 50:1 to 200:1, from 75:1 to 200:1, from 100:1 to 200:1, from 150:1 to 200:1, from 5:1 to 150:1, from 10:1 to 150:1, from 25:1 to 150:1, from 50:1 to 150:1, from 75:1 to 150:1, from 100:1 to 150:1, from 5:1 to 100:1, from 10:1 to 100:1, from 25:1 to 100:1, from 50:1 to 100:1, from 75:1 to 100:1, from 5:1 to 75:1, from 10:1 to 75:1, from 25:1 to 75:1, from 50:1 to 75:1, from 5:1 to 50:1, from 10:1 to 50:1, from 25:1 to 50:1, from 5:1 to 25:1, from 10:1 to 25:1, or from 5:1 to 10:1.

In one or more embodiments, the dehydrogenation catalyst and the feed stream may have a weight hourly space velocity (WHSV) of from 1 to 12 per hour ($h^{-1}$), where WHSV is defined as the weight of the feed stream flow per weight of the dehydrogenation catalyst present in the reaction zone per hour. In embodiments, the dehydrogenation catalyst and the feed stream may have a WHSV of from 1 to 12 $h^{-1}$, from 1 to 10 $h^{-1}$, from 1 to 8 $h^{-1}$, from 1 to 5 $h^{-1}$, from 1 to 3 $h^{-1}$, or from 1 to 2

h$^{-1}$.

In one or more embodiments, the converting at least a portion of the alkanes to alkenes occurs at a temperature that is less than or equal to 750 °C. In embodiments, the converting at least a portion of the alkanes to alkenes occurs at a temperature that is less than or equal to 750 °C, less than or equal to 725 °C, less than or equal to 700 °C, less than or equal to 675 °C, or less than or equal to 650 °C.

In one or more embodiments, the converting at least a portion of the alkanes to alkenes occurs at a pressure that is equal to atmospheric pressure. In embodiments, the converting at least a portion of the alkanes to alkenes occurs at a pressure from 1-20 bar, when measured as an absolute pressure (bara). In embodiments, the converting at least a portion of the alkanes to alkenes occurs at a pressure from 1-20 bara, from 1-15 bara, from 1-10 bara, or from 1-5 bara.

In one or more embodiments, the method of converting alkanes to alkenes may further comprise removing spent dehydrogenation catalyst from the reaction zone and introducing the spent dehydrogenation catalyst into a regeneration zone. In embodiments, the regeneration zone may be part of the reactor. In embodiments, the regeneration zone may the separate from the reactor.

The method of converting alkanes to alkenes may further comprise regenerating the spent dehydrogenation catalyst, thereby forming regenerated dehydrogenation catalyst. Regenerating the dehydrogenation catalyst comprises contacting the dehydrogenation catalyst with a regeneration stream comprising gaseous oxygen, air, or combinations thereof. The regeneration zone is purged with gaseous nitrogen prior to contacting the dehydrogenation catalyst with the regeneration stream. The dehydrogenation catalyst may be regenerated at a temperature of greater than or equal to 650 °C. The dehydrogenation catalyst may be regenerated for a time of greater than or equal to 1 minute (min), greater than 5 min, greater than 10 min, or greater than 30 min. In embodiments, fuel may be combusted to increase the temperature of the dehydrogenation catalyst prior to sending the dehydrogenation catalyst back to the reaction zone.

In embodiments, the method of converting alkanes to alkenes may further comprise regenerating the spent selective hydrogen combustion material, thereby forming regenerated selective hydrogen combustion material. In embodiments, regenerating the selective hydrogen combustion material may comprise contacting the selective hydrogen combustion material with a regeneration stream comprising gaseous oxygen, air, or combinations thereof. In embodiments, the regeneration zone is purged with gaseous nitrogen prior to contacting the selective hydrogen combustion material with the regeneration stream. In embodiments, the selective hydrogen combustion material may be regenerated at a temperature of greater than or equal to 650 °C. In one or more embodiments, the selective hydrogen combustion material may be regenerated for a time of greater than or equal to 1 minute (min), greater than 5 min, greater than 10 min, or greater than 30 min. In embodiments, fuel may be combusted to increase the temperature of the selective hydrogen combustion material prior to sending the selective hydrogen combustion material back to the reaction zone to close heat balance. In embodiments, the selective hydrogen combustion material and the dehydrogenation catalyst may be regenerated together.

In one or more embodiments, the method of converting alkanes to alkenes may further comprise returning regenerated dehydrogenation catalyst to the reaction zone where it is contacted with the feed stream.

**EXAMPLES**

**EXAMPLE 1**

MgGa$_{0.4}$Al$_{0.6}$O$_4$ catalyst was synthesized using a solvothermal approach. Initially, a solution was prepared by dissolving 1.24 g of gallium (III) nitrate hydrate, 0.55 g of magnesium (II) nitrate hexahydrate, 0.43 g of aluminum (III) nitrate nonahydrate, and 2.70 g of urea in 90 mL of methanol. This mixture was stirred magnetically for 30 min in a glass beaker. Subsequently, the solution was introduced into a 125 mL Teflon liner placed within a steel Parr autoclave vessel (Model 4748). The assembly was then subjected to a pre-heated static oven at 168 °C for 10 h, followed by a natural cooling process. Post-cooling, the white precipitate was filtered through a nitrocellulose 0.45 μm membrane, washed with 100 mL of deionized water, and dried in a static oven at 80 °C for 16 h. The final step involved a two-stage thermal treatment: the first at 177 °C for 2 h and the second at 750 °C for 1 h (ramp 5°C/min for both). The resulting solid was recovered, compressed in a press at 7 tons, crushed, and sized to a 40-80 mesh fraction before testing.

**EXAMPLE 2**

MgGa$_{0.5}$Al$_{0.5}$O$_4$ catalyst was synthesized using a solvothermal approach. Initially, a solution was prepared by dissolving 0.83 g of gallium (III) nitrate hydrate, 0.55 g of magnesium (II) nitrate hexahydrate, 0.85 g of aluminum (III) nitrate nonahydrate, and 2.70 g of urea in 90 mL of methanol. This mixture was stirred magnetically for 30 min in a glass beaker. Subsequently, the solution was introduced into a 125 mL Teflon liner placed within a steel Parr autoclave vessel (Model 4748). The assembly was then subjected to a pre-heated static oven at 168 °C for 10 h, followed by a natural cooling process. Post-cooling, the white precipitate was filtered through a nitrocellulose 0.45 μm membrane, washed with 100 mL of deionized water, and dried in a static oven at 80 °C for 16 h. The final step involved a two-stage thermal treatment: the first at 177 °C for 2 h and the second at 750 °C for 1 h (ramp 5°C/min for both). The resulting solid was recovered, compressed in a press at 7 tons, crushed, and sized to a 40-80 mesh fraction before testing. The catalyst's composition, as determined by XRF and XRD, is detailed in Table 1.

## EXAMPLE 3

$Ca_1Ga_{0.5}Al_{0.5}O_4$ was synthesized by the co-precipitation method. In this example, 3.53 g of calcium (II) nitrate tetrahydrate and 5.61 g of aluminum (III) nitrate nonahydrate were dissolved in 150 mL of deionized (DI) water, while another 150 mL of DI water housed 5.44 g of gallium (III) nitrate hydrate. These solutions were combined and stirred rapidly. The pH was adjusted to 8.5 by the gradual addition of ammonia solution (30%). After stirring for over 3 hours, the coprecipitates underwent filtration and extensive washing with DI water. The resultant product was dried at 85 °C overnight, followed by a gradual heating to 160 °C (3 °C/min) for 2 hours. Lastly, the product underwent calcination at 800 °C (3 °C/min) for 4 hours.

## EXAMPLE 4

$ZnGa_2O_4$ was synthesized through the co-precipitation method with careful pH control. In a 30 ml vessel on a heating plate, equipped with a magnetic stir bar, 10 ml of deionized (DI) water and 10 ml of 1.65 M ammonium carbonate in DI water (buffer mixture) were combined. The mixture was heated to 55 °C under continuous stirring, and concurrently, a 0.5 M zinc (II) nitrate solution in DI water (total volume added 5.27 ml), a 0.5 M gallium (III) nitrate solution in DI water (total volume added 8.78 ml), and a base solution of 1.65 M ammonium carbonate in DI water were dropwise added to maintain the mixture's pH at 7.5 +/-0.2. The total time was 15 minutes. The slurry was aged at 55 °C with 700 rpm stirring for 2 hours. The resulting product was separated from the liquid via centrifugation, washed with DI water to eliminate soluble components, and the washed precipitate was dried and calcined in air. The calcination process involved ramping the temperature to 120 °C at 2 °C /min, holding for 2 hours, further ramping to 400 °C at 3 °C /min, holding for 4 hours, homogenizing the product, and re-calcining at 750 °C for 2 hours (ramp rate 5 °C /min). The catalyst underwent compaction (7 tons, 10 minutes), followed by crushing and sieving to achieve a 40-80 mesh size. The catalyst's composition, determined by XRF and XRD, is reported in Table 1.

## EXAMPLE 5

Commercially available inverse spinel $Zn_2TiO_4$ (99%, ALFA AESAR 41673, LOT I13WO26) was calcined on air at 750 °C for 2 hours (ramp rate 5 °C /min). The catalyst was compacted (7 tonn, 10 min) and then crushed and sieved to 40-80 mesh size. The catalyst composition determined by XRF and XRD is reported in Table 1.

## EXAMPLE 6

$CoMgAl_2O_4$ was generated through the utilization of an incipient wetness impregnation method. Commercially available spinel $MgAl_2O_4$ (Puralox Mg 26/100 in the form of 1.5 mm extrudates, BET surface area 109 $m^2$/g, average pore width 15.4 nm, incipient pore volume determined by DI water 0.5 $cm^3$/g) was crushed and sieved to 40-80 mesh size. An impregnation solution was prepared by mixing 0.27 ml of 1M cobalt (II) nitrate in DI water and 0.23 ml of DI water. One g of $MgAl_2O_4$ spinel was impregnated with 0.5 ml of the impregnation solution and homogenized. The material was dried and calcined in air (box oven) using the following program: ramp at 3 °C/min to 150 °C, dwell for 2 hours, ramp to 750 °C at 3 °C/min, dwell for 2 hours, and cool down to room temperature (natural cooling). The catalyst was sieved through 80 mesh size sieve to remove fine particles. The catalyst composition determined by XRF is reported in Table 1.

## EXAMPLE 7

$CrMgAl_2O_4$ was prepared using the incipient wetness impregnation method. Commercially available spinel $MgAl_2O_4$ (Puralox Mg 26/100 in the form of 1.5 mm extrudates, BET surface area 109 $m^2$/g, average pore width 15.4 nm, incipient pore volume determined by DI water 0.5 $cm^3$/g) was crushed and sieved to 40-80 mesh size. An impregnation solution was prepared by mixing 0.299 ml of 1M chromium (III) nitrate in DI water and 0.201 ml of DI water. 1 g of $MgAl_2O_4$ spinel was impregnated with 0.5 ml of the impregnation solution and homogenized. The material was dried and calcined in air (box oven) using the following program: ramp at 3 °C/min to 150 °C, dwell for 2 hours, ramp to 750 °C at 3 °C/min, dwell for 2 hours, and cool down to room temperature (natural cooling). The catalyst was sieved through 80 mesh size sieve to remove fine particles. The catalyst composition determined by XRF is reported in Table 1.

**EXAMPLE 8**

FeMgAl$_2$O$_4$ was prepared using an incipient wetness impregnation method. Commercially available spinel MgAl$_2$O$_4$ (Puralox Mg 26/100 in the form of 1.5 mm extrudates, BET surface area 109 m$^2$/g, average pore width 15.4 nm, incipient pore volume determined by DI water 0.5 cm$^3$/g) was crushed and sieved to 40-80 mesh size. An impregnation solution was prepared by mixing 0.299 ml of 1M iron (III) nitrate in DI water and 0.201 ml of DI water. 1 g of MgAl$_2$O$_4$ spinel was impregnated with 0.5 ml of the impregnation solution and homogenized. The material was dried and calcined in air (box oven) using the following program: ramp at 3 °C/min to 150 °C, dwell for 2 hours, ramp to 750 °C at 3 °C/min, dwell for 2 hours, and cool down to room temperature (natural cooling). The catalyst was sieved through 80 mesh size sieve to remove fine particles. The catalyst composition determined by XRF is reported in Table 1.

**COMPARATIVE EXAMPLE 1**

Commercially available quartz chips (Pyromatics part # 7359-05010) were used to determine background dehydrogenation activity.

**COMPARATIVE EXAMPLE 2**

Commercial Dow propane dehydrogenation catalyst FLUINITY™ was used in the form of 70 micron particles.

**COMPARATIVE EXAMPLE 3**

Commercially available spinel MgAl$_2$O$_4$ (Puralox Mg 26/100 in the form of 1.5 mm extrudates, BET surface area 109 m$^2$/g, average pore width 15.4 nm, incipient pore vlume determined by DI water 0.5 cm$^3$/g) was crushed and sieved to 40-80 mesh size. The catalyst composition determined by XRF is reported in Table 1.

**CATALYST CHARACTERIZATION**

X-RAY FLUORESCENCE (XRF) MEASUREMENTS

Catalyst composition was determined by X-ray Fluorescence (XRF). XRF data were collected at room temperature (RT) with a PANalytical PW4400 spectrometer using an X-ray tube with a rhodium anode. The catalyst compositions are shown in Table 1. Elements that were below the detection limit or not present are represented as blank in the table. Oxygen represented the balance of the elemental composition.

POWDER X-RAY DIFFRACTION (XRD) MEASUREMENTS

The P-XRD measurement was carried out at ambient lab conditions on a Bruker AXS diffractometer D8 Discover with General Area Diffraction Detector System (GADDS) using Cu K$\alpha$ ($\lambda$ = 1.5406 Å). The P-XRD patterns obtained were consistent with spinel structures.

**Table 1: Composition of Catalysts**

|  | Zn (wt.%) | Ti (wt.%) | Mg (wt.%) | Ca (wt.%) | Al (wt.%) | Ga (wt.%) | Cr (wt.%) | Co (wt.%) | Fe (wt.%) |
|---|---|---|---|---|---|---|---|---|---|
| Example 1 |  |  | 7.4 |  | 7.0 | 55.1 |  |  |  |
| Example 2 | - | - | 6.2 | - | 14.5 | 46.1 | - | - | - |
| Example 3 |  |  |  | 5.4 | 13.5 | 47.6 |  |  |  |
| Example 4 | 20.59 | 54.71 | - | - | - | - | - | - | - |
| Example 5 | 55.18 | 18.44 | - | - | - | - | - | - | - |
| Example 6 | - | - | 13.51 | - | 36.81 | - | - | 5.8 | - |
| Example 7 | - | - | 14.04 | - | 38.28 | - | 2.84 | - | - |
| Example 8 | - | - | 13.86 | - | 37.2 | - | - | - | 4.62 |

(continued)

|  | Zn (wt.%) | Ti (wt.%) | Mg (wt.%) | Ca (wt.%) | Al (wt.%) | Ga (wt.%) | Cr (wt.%) | Co (wt.%) | Fe (wt.%) |
|---|---|---|---|---|---|---|---|---|---|
| Comparative Example 3 | - | - | 14.63 | - | 40.01 | - | - | - | - |

## CATALYST PERFORMANCE TESTING

The performance of the catalysts of both in the examples and the comparative examples was assess under both wet and dry conditions. The reaction conditions of both the wet and dry conditions are shown in Table 2.

**Table 2: Reaction Conditions Used to Assess Catalyst Performance**

| Condition | Type | Temperature Range (°C) | Ethane [v.%] | $H_2O$ [v.%] | $N_2$ [v.%] | Catalyst Load [g] | WHSV [$h^{-1}$] | Pressure [bara] (psia) | Catalyst/Ethane (mass/mass) |
|---|---|---|---|---|---|---|---|---|---|
| 1 | Dry test | 650-750 °C | 60 | 0 | 40 | 0.3 | 11.8 | 1.08 (16) | 10-12 |
| 2 | Wet test | 650-750 °C | 60 | 20 | 20 | 0.3 | 11.8 | 1.08 (16) | 10-12 |

The catalysts of the examples and the comparative examples were tested for selectivity and activity in a dual tube fixed-bed reactor. The catalysts were sized to a 40-80 mesh size. The reactor bed comprised 300 milligrams (mg) of catalyst mixed with 1.5 grams (g) of 40-80 mesh size quartz chips. In Comparative Example 1, the catalyst was replaced with quartz chips. The catalysts were evaluated under the four sets of conditions summarized in Table 2.

First, the reactor was purged with nitrogen gas ($N_2$) where the pressure was 1.08 bara (16 psia). The temperature was ramped to 400 °C under $N_2$ flow and then the $N_2$ flow was switched to 60% ethane - 20% $H_2O$ - 20% $N_2$ flow. Water inlet flow was controlled by a high pressure liquid chromatography (HPLC) pump. An evaporator was used to evaporate water to the gas phase. Feed analysis was performed by analyzing the feed gas composition using online gas chromatography (GC).

Then, the reactor was purged with $N_2$ and the temperature was ramped to 650 °C followed by switching from the $N_2$ to air. The catalyst was regenerated at 650 °C for 6 minutes (min) for Conditions 1 and 2, followed by a $N_2$ purge step. Then, the feed composition was directed to the reactor at a controlled time of exposure (25-30 seconds on stream corresponding to catalyst to ethane mass to mass ratio of 12-10). This completes a single cycle at a given temperature set point.

Regeneration of the catalyst after exposure to ethane-containing feed was performed at the same temperature as the corresponding reaction step. Every catalyst was evaluated under four sets of condition specified in Table 2. The wet test were done first, followed by the dry test. For each condition and each temperature set point, 3 cycles (reaction-purge-regeneration) were completed. The average values were then calculated and reported in Table 3 and Table 4.

Alkane conversion and carbon based selectivities are calculated using the following equations:

$$S_j \text{ (Cmol\%)} = [\alpha_j \cdot \eta_{j,\,out} / \sum \alpha_j \cdot \eta_{j,\,out}] \cdot 100$$

(Equation 1)

$$\text{Carbon Balance (\%)} = \sum \alpha_j \cdot \eta_{j,\,out} / \eta_{C_2H_6,\,in} \cdot 100$$

(Equation 2)

$$\text{Ethane Conversion (\%)} = [(\eta_{C_2H_6,\,in} - \eta_{C_2H_6,\,out}) / \eta_{C_2H_6,\,in}] \cdot 100$$

(Equation 3)

$$\text{Reforming Product sel. (Cmol\%)} = S_{CH_4} + S_{CO} + S_{CO_2}$$

(Equation 4)

wherein $\eta$ in is defined as the molar inlet flow of the component (mol/min), $\eta$, out is the molar outlet flow of the component (mol/min), Sj is defined as the carbon based selectivity to product j (%), and $\alpha$j is the number of carbon atoms for product j.

**Table 3: Performance Data for the Examples**

| Example | Condition | T [°C] | Carbon Balance (%) | Ethane Conversion (%) | Ethylene Selectivity (Cmol%) | Reforming Product Selectivity (Cmol%) | Ethylene yield (Cmol%) |
|---|---|---|---|---|---|---|---|
| Example 1 | 1 | 650 | 99.3 | 19.0 | 93.3 | 4.9 | 17.7 |
| Example 1 | 1 | 700 | 96.9 | 23.9 | 89.3 | 8.8 | 21.4 |
| Example 1 | 1 | 725 | 95.5 | 26.2 | 86.6 | 11.8 | 22.7 |
| Example 1 | 1 | 750 | 93.1 | 29.6 | 83.2 | 15.5 | 24.6 |
| Example 1 | 2 | 650 | 101.0 | 6.3 | 87.0 | 13.0 | 5.5 |
| Example 1 | 2 | 700 | 100.6 | 19.3 | 82.5 | 17.3 | 15.9 |
| Example 1 | 2 | 725 | 99.6 | 26.8 | 80.2 | 19.6 | 21.5 |
| Example 1 | 2 | 750 | 97.7 | 36.5 | 76.5 | 23.0 | 27.9 |
| | | | | | | | |
| Example 2 | 1 | 650 | 99.3 | 19.9 | 92.8 | 5.5 | 18.5 |
| Example 2 | 1 | 700 | 94.6 | 30.1 | 87.7 | 10.3 | 26.4 |
| Example 2 | 1 | 725 | 92.1 | 34.7 | 84.3 | 14.0 | 29.3 |
| Example 2 | 1 | 750 | 89.2 | 39.7 | 80.2 | 18.3 | 31.8 |
| Example 2 | 2 | 650 | 101.0 | 4.8 | 88.4 | 11.5 | 4.2 |
| Example 2 | 2 | 700 | 101.3 | 16.2 | 81.4 | 18.5 | 13.2 |
| Example 2 | 2 | 725 | 99.6 | 26.0 | 78.7 | 21.0 | 20.5 |
| Example 2 | 2 | 750 | 97.8 | 35.3 | 75.5 | 24.0 | 26.7 |
| | | | | | | | |
| Example 3 | 1 | 650 | 98.9 | 14.6 | 89.4 | 9.9 | 13.1 |
| Example 3 | 1 | 700 | 95.5 | 22.1 | 82.7 | 16.6 | 18.3 |
| Example 3 | 1 | 725 | 93.6 | 26.0 | 79.1 | 20.1 | 20.6 |
| Example 3 | 1 | 750 | 92.3 | 30.0 | 76.7 | 22.4 | 23.0 |
| Example 3 | 2 | 650 | 101.5 | 5.6 | 67.2 | 32.5 | 3.8 |
| Example 3 | 2 | 700 | 102.4 | 16.8 | 65.0 | 34.5 | 10.9 |
| Example 3 | 2 | 725 | 101.5 | 26.7 | 65.2 | 34.2 | 17.4 |
| Example 3 | 2 | 750 | 100.0 | 38.3 | 64.1 | 35.0 | 24.5 |
| | | | | | | | |
| Example 4 | 1 | 650 | 99.3 | 12.1 | 94.9 | 6.6 | 11.5 |
| Example 4 | 1 | 700 | 98.2 | 11.9 | 90.5 | 11.0 | 10.8 |
| Example 4 | 1 | 725 | 98.1 | 13.3 | 88.6 | 13.5 | 11.8 |
| Example 4 | 1 | 750 | 97.9 | 16.9 | 87.6 | 15.3 | 14.8 |
| Example 4 | 2 | 650 | 101.5 | 22.4 | 64.0 | 25.0 | 14.3 |

(continued)

| Example | Condition | T [°C] | Carbon Balance (%) | Ethane Conversion (%) | Ethylene Selectivity (Cmol%) | Reforming Product Selectivity (Cmol%) | Ethylene yield (Cmol%) |
|---|---|---|---|---|---|---|---|
| Example 4 | 2 | 700 | 99.5 | 29.1 | 62.8 | 27.2 | 18.3 |
| Example 4 | 2 | 725 | 98.8 | 29.1 | 66.0 | 26.4 | 19.2 |
| Example 4 | 2 | 750 | 98.6 | 30.1 | 73.6 | 23.7 | 22.1 |
| | | | | | | | |
| Example 5 | 1 | 650 | 100.5 | 2.6 | 99.6 | 12.0 | 2.6 |
| Example 5 | 1 | 700 | 100.8 | 8.1 | 97.3 | 13.3 | 7.9 |
| Example 5 | 1 | 725 | 100.8 | 12.3 | 97.8 | 14.6 | 12.1 |
| Example 5 | 1 | 750 | 100.8 | 17.7 | 96.7 | 16.2 | 17.2 |
| Example 5 | 2 | 650 | 101.2 | 1.5 | 91.3 | 19.9 | 1.4 |
| Example 5 | 2 | 700 | 97.1 | 9.0 | 90.0 | 21.4 | 8.1 |
| Example 5 | 2 | 725 | 101.2 | 8.3 | 90.1 | 22.6 | 7.5 |
| Example 5 | 2 | 750 | 101.3 | 13.8 | 89.7 | 23.8 | 12.4 |
| | | | | | | | |
| Example 6 | 1 | 650 | 98.9 | 19.8 | 78.6 | 20.6 | 15.5 |
| Example 6 | 1 | 700 | 96.4 | 27.5 | 89.4 | 9.4 | 24.6 |
| Example 6 | 1 | 725 | 96.1 | 23.6 | 89.7 | 9.0 | 21.2 |
| Example 6 | 1 | 750 | 96.5 | 19.8 | 90.5 | 8.3 | 17.9 |
| Example 6 | 2 | 650 | 100.2 | 7.9 | 79.5 | 20.3 | 6.3 |
| Example 6 | 2 | 700 | 100.4 | 19.2 | 71.3 | 28.5 | 13.7 |
| Example 6 | 2 | 725 | 101.0 | 26.0 | 66.3 | 33.4 | 17.2 |
| Example 6 | 2 | 750 | 102.0 | 30.9 | 62.9 | 36.9 | 19.4 |
| | | | | | | | |
| Example 7 | 1 | 650 | 93.1 | 27.8 | 65.9 | 31.8 | 18.3 |
| Example 7 | 1 | 700 | 84.1 | 42.6 | 72.5 | 25.5 | 30.9 |
| Example 7 | 1 | 725 | 82.4 | 46.9 | 75.8 | 22.2 | 35.6 |
| Example 7 | 1 | 750 | 81.8 | 49.2 | 78.3 | 20.1 | 38.5 |
| Example 7 | 2 | 650 | 102.0 | 15.1 | 72.0 | 27.2 | 10.9 |
| Example 7 | 2 | 700 | 103.6 | 32.1 | 56.3 | 43.1 | 18.0 |
| Example 7 | 2 | 725 | 105.9 | 43.2 | 47.1 | 52.5 | 20.3 |
| Example 7 | 2 | 750 | 101.0 | 54.5 | 45.7 | 53.5 | 24.9 |
| | | | | | | | |
| Example 8 | 1 | 650 | 100.1 | 8.0 | 92.6 | 7.2 | 7.4 |
| Example 8 | 1 | 700 | 85.4 | 31.5 | 91.6 | 8.1 | 28.9 |
| Example 8 | 1 | 725 | 74.5 | 42.9 | 89.8 | 9.5 | 38.5 |
| Example 8 | 1 | 750 | 63.4 | 55.3 | 87.2 | 11.9 | 48.2 |
| Example 8 | 2 | 650 | 99.7 | 3.1 | 91.3 | 8.7 | 2.8 |
| Example 8 | 2 | 700 | 100.0 | 8.7 | 88.4 | 11.3 | 7.7 |

(continued)

| Example | Condition | T [°C] | Carbon Balance (%) | Ethane Conversion (%) | Ethylene Selectivity (Cmol%) | Reforming Product Selectivity (Cmol%) | Ethylene yield (Cmol%) |
|---|---|---|---|---|---|---|---|
| Example 8 | 2 | 725 | 100.1 | 14.6 | 86.3 | 13.1 | 12.6 |
| Example 8 | 2 | 750 | 100.3 | 23.8 | 83.2 | 15.9 | 19.8 |

**Table 4: Performance Data for the Comparative Examples**

| Example | Condition | T [°C] | Carbon Balance (%) | Ethane Conversion (%) | Ethylene Selectivity (Cmol%) | Reforming Product Selectivity (Cmol%) | Ethylene yield (Cmol%) |
|---|---|---|---|---|---|---|---|
| C. Ex. 1 | 1 | 650 | 99.9 | 0.3 | 99.3 | 0.7 | 0.3 |
| C. Ex. 1 | 1 | 700 | 100.5 | 1.5 | 99.0 | 0.9 | 1.5 |
| C. Ex. 1 | 1 | 725 | 100.6 | 4.6 | 98.7 | 1.0 | 4.5 |
| C. Ex. 1 | 1 | 750 | 101.0 | 11.0 | 98.3 | 1.3 | 10.8 |
| C. Ex. 1 | 2 | 650 | 100.1 | 0.1 | 99.4 | 0.6 | 0.1 |
| C. Ex. 1 | 2 | 700 | 101.3 | 1.1 | 99.1 | 0.8 | 1.1 |
| C. Ex. 1 | 2 | 725 | 102.1 | 4.0 | 98.8 | 0.9 | 4.0 |
| C. Ex. 1 | 2 | 750 | 102.4 | 10.5 | 98.3 | 1.2 | 10.3 |
| | | | | | | | |
| C. Ex. 2 | 1 | 650 | 100.6 | 26.4 | 99.6 | 0.1 | 26.3 |
| C. Ex. 2 | 1 | 700 | 100.6 | 33.3 | 98.8 | 0.6 | 32.9 |
| C. Ex. 2 | 1 | 725 | 100.2 | 37.0 | 98.3 | 0.9 | 36.4 |
| C. Ex. 2 | 1 | 750 | 99.0 | 41.1 | 97.6 | 1.5 | 40.1 |
| C. Ex. 2 | 2 | 650 | 98.1 | 3.9 | 90.0 | 9.9 | 3.5 |
| C. Ex. 2 | 2 | 700 | 101.1 | 2.9 | 91.9 | 7.5 | 2.7 |
| C. Ex. 2 | 2 | 725 | 99.8 | 7.1 | 93.1 | 6.0 | 6.6 |
| C. Ex. 2 | 2 | 750 | 101.3 | 12.4 | 94.4 | 4.2 | 11.7 |
| | | | | | | | |
| C. Ex. 3 | 1 | 650 | 99.7 | 1.7 | 95.0 | 5.0 | 1.6 |
| C. Ex. 3 | 1 | 700 | 100.4 | 9.3 | 97.4 | 2.4 | 9.0 |
| C. Ex. 3 | 1 | 725 | 98.9 | 28.3 | 96.0 | 2.5 | 27.2 |
| C. Ex. 3 | 1 | 750 | 96.4 | 35.8 | 93.5 | 4.8 | 33.5 |
| C. Ex. 3 | 2 | 650 | 101.5 | <0.1 | 98.3 | 1.7 | <0.1 |
| C. Ex. 3 | 2 | 700 | 101.3 | 0.3 | 87.9 | 11.8 | 0.2 |
| C. Ex. 3 | 2 | 725 | 101.7 | 2.6 | 90.2 | 9.0 | 2.4 |
| C. Ex. 3 | 2 | 750 | 102.0 | 8.5 | 91.4 | 7.6 | 7.8 |

Examples 1-8 comprise spinel catalysts containing dehydrogenation active elements. Specifically, Examples 1-5 are bulk-type catalysts with dehydrogenation active elements in the A or B site of the spinel $AB_2O_4$ structure. Examples 6-8 are supported-type catalysts where the spinel structure has a dual function. More specifically, in Examples 6-8, the spinel structure acts as a support for metal nanoparticles and also functions as a dehydrogenation active element as part of the spinel structure.

As shown in Table 3, Examples 1-8 demonstrate substantial activity above the background dehydrogenation activity (as measured in Comparative Example 1 by using quartz chips in lieu of a catalyst). Additionally, the catalysts of Examples 1-8 also demonstrate steam tolerance when compared with Comparative Example 2. Furthermore, the catalysts of Examples 1-8 show that spinel catalysts with dehydrogenation elements in the A or B site of $AB_2O_4$ (or $A_2BO_4$ in the case of Example 6), have a higher ethylene selectivity than spinel catalysts that lack dehydrogenation active elements, such as that of Comparative Example 3.

The subject matter of the present disclosure has been described in detail and by reference to specific embodiments. It should be understood that any detailed description of a component or feature of an embodiment does not necessarily imply that the component or feature is essential to the particular embodiment or to any other embodiment. Further, it should be apparent to those skilled in the art that various modifications and variations can be made to the described embodiments without departing from the spirit and scope of the claimed subject matter.

It is noted that one or more of the following claims utilize the term "wherein" as a transitional phrase. For the purposes of defining the present technology, it is noted that this term is introduced in the claims as an open-ended transitional phrase that is used to introduce a recitation of a series of characteristics of the structure and should be interpreted in like manner as the more commonly used open-ended preamble term "comprising."

It should be understood that where a first component is described as "comprising" a second component, it is contemplated that, in embodiments, the first component "consists" or "consists essentially of" that second component. It should further be understood that where a first component is described as "comprising" a second component, it is contemplated that, in embodiments, the first component comprises at least 10%, at least 20%, at least 30%, at least 40%, at least 50%, at least 60%, at least 70%, at least 80%, at least 90%, at least 95%, or even at least 99% that second component (where % can be weight % or molar %).

It is also noted that recitations herein of "at least one" component, element, etc., should not be used to create an inference that the alternative use of the articles "a" or "an" should be limited to a single component, element, etc.

## Claims

1. A method for converting alkanes to alkenes, the method comprising:

   introducing a feed stream comprising alkanes to a reaction zone comprising steam and a dehydrogenation catalyst, the dehydrogenation catalyst comprising:

   a component having a spinel structure; and
   a metal catalyst component selected from the group consisting of chromium, gallium, iron, cobalt, and combinations thereof; and

   converting at least a portion of the alkanes to alkenes, thereby yielding a product stream comprising alkanes, alkenes, and hydrogen.
   wherein the dehydrogenation catalyst does not require a gaseous oxidant in the feed stream or as a co-feed to catalyze conversion of alkanes to alkenes.

2. The method of claim 1, wherein the metal catalyst component is part of the spinel structure.

3. The method of claim 1, wherein the metal catalyst component is supported by the spinel structure.

4. The method of claim 3, wherein the spinel structure is impregnated with the metal catalyst component.

5. The method of any of the preceding claims, wherein the dehydrogenation catalyst comprises a promoter selected from the group consisting of platinum, tin, boron, silicon, zirconia, and combinations thereof.

6. The method of any of the preceding claims, further comprising combusting at least a portion of hydrogen within the product stream to yield steam.

7. The method of any of the preceding claims, wherein the dehydrogenation catalyst measures an alkene selectivity greater than or equal to 40 Cmol%.

8. The method of any of the preceding claims, wherein the dehydrogenation catalyst retains at least 1.1 times a background dehydrogenation activity when an atmosphere within the reaction zone comprises greater than or equal to 20 v.% steam.

9.  The method of any of the preceding claims, wherein the component having a spinel structure has a formula $AB_2O_4$, wherein

    A is a metal selected from the group consisting of magnesium, calcium, zinc, and combinations thereof, and
    B is selected from the group consisting of aluminium, chromium, gallium, iron, titanium, zinc and titanium, and combinations thereof.

10. The method of any of the preceding claims, wherein the dehydrogenation catalyst comprises:

    1 wt.% to 30 wt.% of the metal catalyst component; and
    70 wt.% to 99 wt.% of the component having a spinel structure;

    wherein the weight percent is based on a total weight of the dehydrogenation catalyst.

11. The method of any of the preceding claims, wherein the dehydrogenation catalyst and the feed stream have a mass to mass ratio that is from 5:1 to 200:1.

12. The method of any of the preceding claims, wherein the converting at least a portion of the alkanes to alkenes occurs at a temperature that is less than or equal to 750 °C, a pressure that is from 1 bara to 20 bara, and a WHSV that is from 1 $h^{-1}$ to 12 $h^{-1}$.

13. The method of any of the preceding claims, wherein the method further comprises:

    removing spent dehydrogenation catalyst from the reaction zone;
    introducing the spent dehydrogenation catalyst into a regeneration zone;
    regenerating the spent dehydrogenation catalyst, thereby forming regenerated dehydrogenation catalyst; and
    returning the regenerated dehydrogenation catalyst to the reaction zone where it is contacted with the feed stream.

14. The method of any of the preceding claims, further comprising contacting the feed stream comprising alkanes with a selective hydrogen combustion material, wherein the dehydrogenation catalyst and the selective hydrogen combustion material are both present in the reaction zone.

15. The method of claim 14, wherein the method further comprises:

    removing spent selective hydrogen combustion material from the reaction zone;
    introducing the spent selective hydrogen combustion material into a regeneration zone;
    regenerating the spent selective hydrogen combustion material, thereby forming regenerated selective hydrogen combustion material; and
    returning the selective hydrogen combustion material to the reaction zone where it is contacted with the feed stream.

Europäisches
Patentamt

European
Patent Office

Office européen
des brevets

## EUROPEAN SEARCH REPORT

**Application Number**

EP 24 38 2166

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 105 363 455 A (CHINA PETROLEUM & CHEM CORP ET AL.) 2 March 2016 (2016-03-02) | 1-15 | INV.<br>C07C5/333 |
| Y | * paragraph [0023] *<br>* examples *<br>* tables 1-3 * | 1-15 | C07C5/48<br>C07C11/02 |
| X | GB 1 550 873 A (SHELL INT RESEARCH) 22 August 1979 (1979-08-22) | 1-15 | |
| Y | * claims * | 1-15 | |
| X | US 4 372 879 A (GERMAINE GILBERT R [FR] ET AL) 8 February 1983 (1983-02-08) | 1-15 | |
| Y | * column 2, lines 29-52 *<br>* column 3, lines 60-63 *<br>* column 4, lines 23-26 * | 1-15 | |
| Y | WO 93/12879 A1 (EXXON RESEARCH ENGINEERING CO [US]) 8 July 1993 (1993-07-08)<br>* table I * | 1-15 | |
| Y,D | WO 2018/232133 A1 (UNIV NORTH CAROLINA STATE [US]) 20 December 2018 (2018-12-20)<br>* claims; examples *<br>* paragraph [0023] * | 1-15 | **TECHNICAL FIELDS SEARCHED (IPC)**<br><br>C07C<br>B01J |

The present search report has been drawn up for all claims

| Place of search | Date of completion of the search | Examiner |
|---|---|---|
| The Hague | 12 July 2024 | Kardinal, Siegmar |

EPO FORM 1503 03.82 (P04C01)

**ANNEX TO THE EUROPEAN SEARCH REPORT**
**ON EUROPEAN PATENT APPLICATION NO.**
                                                EP 24 38 2166

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2024

| Patent document cited in search report | | Publication date | Patent family member(s) | | Publication date |
|---|---|---|---|---|---|
| CN 105363455 | A | 02-03-2016 | NONE | | |
| GB 1550873 | A | 22-08-1979 | CA | 1072987 A | 04-03-1980 |
| | | | DE | 2648443 A1 | 05-05-1977 |
| | | | FR | 2329620 A1 | 27-05-1977 |
| | | | GB | 1550873 A | 22-08-1979 |
| | | | IT | 1069010 B | 21-03-1985 |
| | | | JP | S603289 B2 | 26-01-1985 |
| | | | JP | S5253803 A | 30-04-1977 |
| | | | NL | 7611828 A | 02-05-1977 |
| US 4372879 | A | 08-02-1983 | AU | 543768 B2 | 02-05-1985 |
| | | | BE | 891515 A | 18-06-1982 |
| | | | BR | 8108327 A | 05-10-1982 |
| | | | CA | 1171053 A | 17-07-1984 |
| | | | DE | 3150820 A1 | 26-08-1982 |
| | | | ES | 8300660 A1 | 01-11-1982 |
| | | | FR | 2497192 A1 | 02-07-1982 |
| | | | GB | 2091757 A | 04-08-1982 |
| | | | IN | 157490 B | 12-04-1986 |
| | | | IT | 1140365 B | 24-09-1986 |
| | | | JP | H0222738 B2 | 21-05-1990 |
| | | | JP | S57130927 A | 13-08-1982 |
| | | | KR | 890001300 B1 | 29-04-1989 |
| | | | MX | 159421 A | 30-05-1989 |
| | | | NL | 8105771 A | 16-07-1982 |
| | | | US | 4372879 A | 08-02-1983 |
| | | | US | 4395579 A | 26-07-1983 |
| | | | ZA | 818860 B | 24-11-1982 |
| WO 9312879 | A1 | 08-07-1993 | NONE | | |
| WO 2018232133 | A1 | 20-12-2018 | US | 2020215515 A1 | 09-07-2020 |
| | | | US | 2021213424 A1 | 15-07-2021 |
| | | | US | 2023415124 A1 | 28-12-2023 |
| | | | WO | 2018232133 A1 | 20-12-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62725504
- US 62725508
- US 5430209 A
- US 7122495 B
- WO 2018232133 A